(19) Europäisches Patentamt
European Patent Office
Office européen des brevets

(11) Numéro de publication : 0 309 340 B1

(12) FASCICULE DE BREVET EUROPEEN

(45) Date de publication du fascicule du brevet : 29.05.91 Bulletin 91/22

(51) Int. Cl.[5] : C07D 317/16, C07D 317/26, C07C 43/313

(21) Numéro de dépôt : 88402374.8

(22) Date de dépôt : 21.09.88

# (54) Nouveaux dérivés du nonatriène-1,3,5, leur préparation et leur emploi.

(30) Priorité : 22.09.87 FR 8713055

(43) Date de publication de la demande : 29.03.89 Bulletin 89/13

(45) Mention de la délivrance du brevet : 29.05.91 Bulletin 91/22

(84) Etats contractants désignés : AT BE CH DE ES FR GB GR IT LI LU NL SE

(56) Documents cités : EP-A- 0 012 390

(73) Titulaire : RHONE-POULENC SANTE
20, avenue Raymond Aron
F-92160 Antony (FR)

(72) Inventeur : Duhamel, Lucette
32 rue Jacques Boutrolle
F-76130 Mont-Saint-Aignan (FR)
Inventeur : Duhamel, Pierre
32 rue Jacques Boutrolle
F-76130 Mont-Saint-Aignan (FR)

(74) Mandataire : Le Pennec, Magali et al
RHONE-POULENC SANTE, Service Brevets,
20 Avenue Raymond Aron
F-92165 Antony Cédex (FR)

EP 0 309 340 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un nouveau dérivé du nonatriène-1,3,5 de formule générale :

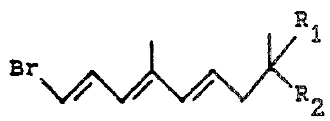

(I)

sa préparation et son emploi.

Dans la formule générale (I), les symboles $R_1$ et $R_2$ forment ensemble avec l'atome de carbone auquel ils sont liés un groupement carbonyl ou bien représentent chacun un radical alkoxy contenant 1 à 4 atomes de carbone ou forment ensemble un radical alkylènedioxy.

Selon la présente invention, les produits de formule générale (I) peuvent être obtenus par action d'un dérivé du butadiène-1,3 de formule générale :

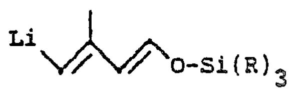

(II)

dans laquelle R représente un radical alkyle contenant 1 à 4 atomes de carbone ou un radical phényle, préparé in situ par échange halogène-métal avec le dérivé bromé correspondant, sur un produit de formule générale :

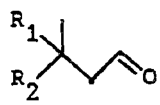

(III)

dans laquelle $R_1$ et $R_2$ représentent chacun un radical alkoxy ou forment ensemble un radical alkylènedioxy suivie de l'action d'un halogénure d'halométhyltriphénylphosphonium sur l'oxocétal du méthyl-3 oxo-7 octadiène-2,4 al ainsi obtenu.

Généralement, le produit de formule générale (III) est ajouté au produit de formule générale (II) préparé in situ par action d'un dérivé organolithien, tel que le tertiobutyllithium, à une température inférieure à –20°C, de préférence inférieure à –50°C, en opérant dans un solvant organique anhydre choisi parmi les éthers tels que l'éther éthylique ou le tétrahydrofuranne et les hydrocarbures aliphatiques ou aromatiques. L'oxocétal du méthyl-3 oxo-7 octadiène-2,4 al est obtenu après hydrolyse en milieu acide du mélange réactionnel en opérant généralement à une température voisine de 20°C.

Généralement, l'action de l'halogénure de l'halométhyltriphénylphosphonium, tel que le bromure de brométhyltriphénylphosphonium, sur l'oxocétal du méthyl-3 oxo-7 octadiène-2,4 al est effectuée en présence d'un alcoolate métallique, tel que le tertiobutylate de potassium, en opérant dans un solvant organique anhydre tel que le tétrahydrofuranne.

Le bromo-1 méthyl-2 trialkylsilyloxy-4 butadiène-1,3, précurseur du produit de formule générale (II) peut être obtenu par action d'un halogénotrialkylsilane, tel que le chlorotriméthylsilane ou le bromotriméthylsilane, sur le bromo-4 méthyl-3 butène-2 al en opérant dans un solvant organique tel que le pentane et l'acétonitrile en présence d'une base organique telle que la triéthylamine à une température voisine de 20°C.

La bromo-4 méthyl-3 butène-2 al peut être obtenu par action du N-bromosuccinimide sur un méthyl-3 trialkylsilyloxy-1 butadiène en milieu hydro-alcoolique à une température inférieure à 10°C, suivie d'hydrolyse en milieu acide.

Un méthyl-3 trialkylsilyloxy-1 butadiène peut être obtenu par action d'un halogénotrialkylsilane sur le prénal en opérant dans un solvant organique tel que l'éther éthylique en présence d'une base organique telle que la triéthylamine.

Le produit de formule générale (III) peut être obtenu par acétalisation de la fonction cétone de l'acétylacétate d'éthyle suivie de la réduction de l'acétal obtenu en alcool au moyen par exemple d'hydrure de lithium et d'aluminium puis oxydation de l'alcool en cétocétal aldéhyde de formule (III) par exemple au moyen du mélange $CrO_3$-pyridine.

Les produits de formule générale (I) sont des intermédiaires particulièrement utiles en synthèse terpénique.

Par exemple, les produits de formule générale (I) peuvent être condensés sur la méthylhepténone pour

conduire à l'acétal de l'hydroxy-10 triméthyl-6,10,14 pentadécatétraène-4,6,8,13 one-2 qui, après hydrolyse, déshydratation et réduction, fournit la triméthyl-6,10,14 pentadécanone-2 qui, après condensation sur le triméthylsilylvinyllithium, fournit le tétraméthyl-3,7,11,15 hexadécène-2 al qui, après réduction, donne le phytol qui permet, par condensation sur la triméthylhydroquinone, d'obtenir la vitamine E.

La condensation d'un produit de formule générale (I) sur la méthylhepténone s'effectue dans les conditions décrites précédemment pour la condensation d'un produit de formule générale (III) sur un produit de formule générale (II) après réaction d'échange halogène-métal.

La déshydratation et l'hydrolyse de l'hydroxyacétal ainsi obtenu s'effectue par chauffage en milieu hydroacétonique en milieu acide.

Les exemples suivants montrent comment l'invention peut être mise en pratique.

EXEMPLE 1

Dans un ballon de 50 cm3, on introduit, sous atmosphère d'argon, 11 cm3 d'éther éthylique anhydre et 0,9 g de bromo-1 méthyl-2 triméthylsilyloxy-4 butadiène-1,3. On refroidit à –70°C puis on ajoute en 10 minutes, 3,25 cm3 d'une solution de tertiobutyllithium 1,65 M dans le pentane. On agite pendant 100 minutes à –70°C puis on ajoute 0,4 g d'éthylènedioxy-3,3 butanal en solution dans 4 cm3 d'éther éthylique anhydre. On laisse la température remonter à –30°C et agite pendant 30 minutes. On agite ensuite pendant 20 minutes à –20°C puis refroidit à –60°C. On ajoute 11,5 cm3 d'acide chlorhydrique N en 20 minutes. On laisse la température remonter au voisinage de 20°C. Après reprise du mélange réactionnel par l'éther et l'eau et séchage des phases organiques sur sulfate de magnésium, on obtient, après chromatographie éclair, 0,33 g d'éthylènedioxy-7,7 méthyl-3 octadiène-2,4 al dont les caractéristiques sont les suivantes :

– Spectre de résonance magnétique nucléaire du proton (60 MHz ; $CCl_4$ ; déplacements chimiques en ppm ; constantes de couplage en Hz) :

10 (d, 1H, 8,5 Hz) ; 6,2 (m, 2H) ; 5,8 (d, 1H, 8,5) ; 3,9 (s, 4H) ; 2,4 (d, 2H, 3,3 Hz) ; 2,2 (s, 2, 4H) ; 2,0 (s, 0, 6H) ; 1,1 (s, 3H)

– Spectre infra-rouge : 2960, 1665, 1595, 1380, 1205, 1120 et 1050 $cm^{-1}$.

Le rendement est de 55%.

Le rapport des formes E/Z est 80/20.

Dans un ballon de 50 cm3, on introduit 22 cm3 de tétrahydrofuranne anhydre et 1,7 g de bromure de bromométhyl-triphénylphosphonium (3,9 mmoles). On refroidit à –70°C puis on ajoute en 10 minutes 0,44 g de tertiobutylate de potassium. On agite pendant 1 heure 30 minutes puis on ajoute 0,5 g d'éthylènedioxy-7,7 méthyl-3 octadiène-2,4 al en solution dans 3 cm3 de tétrahydrofuranne en 10 minutes. On laisse la température remonter à 10°C puis on agite pendant 2 heures 20 minutes. On ajoute rapidement 12 cm3 d'eau puis agite pendant 15 minutes. Après extraction, les phases organiques sont séchées sur sulfate de magnésium. Après évaporation des solvants, le produit obtenu est placé dans un mortier et est repris par de l'éther de pétrole : l'oxyde de triphénylphosphine qui précipite est broyé jusqu'à blanchissement puis séparé par filtration. Après évaporation de l'éther de pétrole, on obtient 0,49 g d'éthylènedioxy-8,8 bromo-1 méthyl-4 nonatriène-1,3,5 dont les caractéristiques sont les suivantes :

– Spectre de résonance magnétique nucléaire du proton (60 MHz ; $CCl_4$) :

5,4-6,6 (m, 5H) ; 3,95 (s, 4H) ; 2,45 (d, 2H, 6,4 Hz) ; 1,85 (s, 3H) ; 1,25 (s, 3H).

– Spectre infra-rouge (film) : 2980, 1445, 1380, 1105 et 1050 $cm^{-1}$.

Le rendement est de 70%.

Le bromo-1 méthyl-2 triméthylsilyloxy-4 butadiène peut être préparé de la manière suivante :

Dans un ballon tricol de 500 cm3, on introduit, sous atmosphère d'argon, 120 cm3 de pentane, 120 cm3 d'acétonitrile, 9 g de bromo-4 méthyl-3 butène-2 al (107 mmoles) et 13 g de triéthylamine. On maintient la température à 0°C, puis on ajoute 19,6 g de bromotriméthylsilane en 20 minutes. On agite à une température voisine de 20°C pendant 2 jours. La solution de pentane est prélevée à la seringue puis remplacée par une quantité équivalente de pentane. L'opération est répétée 4 fois. Les différentes solutions pentaniques sont rassemblées et la pentane est éliminé en évitant toute entrée d'air. Le résidu obtenu est purifié par distillation. On obtient ainsi 6,5 g de bromo-1 méthyl-2 triméthylsilyloxy-4 butadiène ($E.E._{0,25}$ = 59°C).

Le rendement est de 50%.

Le bromo-4 méthyl-3 butène-2 al peut être préparé de la manière suivante :

A un mélange de 160 cm3 de méthanol et de 16 cm3 d'eau maintenu à 0°C, on ajoute rapidement 8 g de méthyl-3 triméthylsilyloxy-1 butadiène (51,2 mmoles) et aussitôt 9,12 g de N-bromosuccinimide finement pulvérisé en 25 minutes et en maintenant la température inférieure à 50°C. On agite pendant 15 minutes à 3°C puis évapore le méthanol. Le résidu est repris par 150 cm3 puis 2 fois 50 cm3 d'éther de pétrole. On évapore l'éther de pétrole, puis reprend par 200 cm3 d'éther éthylique. On ajoute 15 cm3 d'acide chlorhydrique N et

agite pendant 30 minutes à 3°C. Après extraction, séchage des phases organiques sur sulfate de magnésium et évaporation des solvants, on obtient 7,1 g de bromo-4 méthyl-3 butène-2 al avec un rendement de 85%.

Le méthyl-3 triméthylsilyloxy-1 butadiène peut être préparé de la manière suivante :

Dans un ballon de 250 cm3, on introduit, sous atmosphère d'argon, 70 cm3 d'éther éthylique anhydre, 35 g de prénal distillé (0,42 mole), 45 g de triéthylamine distillée et 0,5 g de chlorure de zinc séché sur anhydride phosphorique. On ajoute en 30 minutes 57 cm3 de chlorotriméthylsilane. On chauffe au reflux pendant 25 heures. Après refroidissement, on ajoute 75 cm3 de pentane et agite pendant 15 minutes. Le chlorhydrate de triéthylamine est séparé par filtration et lavé avec 300 cm3 de pentane. Après évaporation des solvants, le résidu est distillé. On obtient ainsi 50 g de méthyl-3 triméthylsilyloxy-1 butadiène ($P.E._{13}$ = 43°C).

Le rendement est de 77%.

L'éthylènedioxy-3,3 butanal peut être préparé de la manière suivante :

A 150 cm3 de dichlorométhane, on ajoute 7,2 g de pyridine et 4,6 g d'oxyde de chrome ($CrO_3$) par petites fractions en 10 minutes. Après 15 minutes d'agitation à une température voisine de 20°C, on ajoute rapidement 0,85 g d'éthylènedioxy-2,2 hydroxy-4 butane (6,4 mmoles) en solution dans 2 cm3 de dichlorométhane. Après 15 minutes d'agitation à 20°C, le précipité noir, visqueux est séparé par filtration sur silice et lavé à l'éther. Après évaporation des solvants, on obtient 0,59 g d'éthylènedioxy-3,3 butanal ($P.E._{0,4}$ = 35°C).

Le rendement est de 70%.

L'éthylènedioxy-2,2 hydroxy-4 butane peut être préparé de la manière suivante :

Dans 200 cm3 d'éther éthylique anhydre, on ajoute par petites fractions 2 g d'hydrure de lithium et d'aluminium (51 mmoles). On refroidit à 0°C puis on ajoute en 10 minutes 3 g d'éthylènedioxy-3,3 butanoate d'éthyle (17 mmoles) en solution dans 10 cm3 d'éther éthylique anhydre. On agite pendant 2 heures 30 minutes à une température voisine de 20°C puis hydrolyse par addition de 12 cm3 d'une solution saturée de sulfate de sodium. Après 2 heures, le précipité blanc est séparé par filtration. Les phases organiques sont séchées sur sulfate de magnésium. On obtient ainsi 2,22 g d'éthylènedioxy-2,2 hydroxy-4 butane.

Le rendement est de 97%.

L'éthylènedioxy-3,3 butanoate d'éthyle peut être préparé de la manière suivante :

Dans un ballon muni d'un appareil Dean-Stark, on introduit 50 cm3 de benzène, 30 g d'acétylacétate d'éthyle (231 mmoles), 16 g d'éthylèneglycol puis 0,5 g d'acide p.toluènesulfonique. On chauffe au reflux en distillant l'azéotrope en 2 heures 30 minutes. Après élimination du benzène, on obtient 37 g d'éthylènedioxy-3,3 butanoate d'éthyle ($P.E._{12}$ = 98°C).

Le rendement est de 90%.

EXEMPLE 2

Dans 8 cm3 d'éther éthylique, on introduit, sous atmosphère d'argon, 0,3 g d'éthylènedioxy-8,8 bromo-1 méthyl-4 nonatriène-1,3,5 (1 mmole) et on refroidit à −70°C. En 10 minutes, on ajoute 1,2 cm3 d'une solution de tertiobutyllithium 1,65 M dans le pentane. On agite pendant 1 heure 50 minutes puis on ajoute en 10 minutes 0,1 g de méthylhepténone en solution dans 3 cm3 d'éther éthylique anhydre. On laisse la température remonter au voisinage de 20°C en 1 heure puis agite encore pendant 25 minutes. On refroidit à −10°C puis on ajoute 2,6 cm3 d'une solution de bicarbonate de sodium à 5%. Après extraction à l'éther éthylique et séchage des phases organiques sur sulfate de magnésium, puis chromatographie éclair, on obtient, avec un rendement de 58%, 0,15 g d'éthylènedioxy-2,2 hydroxy-10 triméthyl-6,10,14 pentadécatétraène-4,6,8,13 dont les caractéristiques sont les suivantes :

– Spectre de résonance magnétique nucléaire du proton (60 MHz ; $CDCl_3$) :

5,3-7 (m, 5H) ; 5,1 (t, 1H, 6,5 Hz) ; 3,95 (s, 4H) ; 2,43 (d, 2H, 7,2 Hz) ; 2,05 (m, 1H) ; 1,05-1,95 (m, 19H)

– Spectre infra-rouge (film) : 3495, 2930, 1450, 1375, 1105 et 1050 $cm^{-1}$.

On chauffe au reflux pendant 3 minutes, 0,11 g de l'hydroxy-acétal obtenu ci-dessus dans 5 cm3 d'acétone. On ajoute alors 0,55 cm3 d'acide chlorhydrique N et maintient au reflux pendant 20 minutes. Après refroidissement, le mélange réactionnel est versé dans 30 cm3 d'une solution aqueuse de carbonate de sodium à 5%. Après extraction à l'éther, séchage des phases organiques sur sulfate de magnésium et évaporation des solvants, on obtient 0,08 g de triméthyl-6,10,14 pentadécaène-3,5,7,13 one-2.

Le rendement est de 90%.

Dans un ballon de 100 cm3, on introduit 20 cm3 d'éthanol 0,4 g de triméthyl-6,10,14 pentadécaène-3,5,7,9,13 one-2 et 0,2 g de palladium sur charbon à 5% en poids de palladium. On purge à l'argon puis met sous atmosphère d'hydrogène. Après 2 heures d'agitation à une température voisine de 20°C, le catalyseur est séparé par filtration et lavé par de l'éthanol. Après concentration à sec des phases éthanoliques, on obtient 0,35 g de triméthyl-6,10,14 pentadécanone-2 qui est caractérisée par son spectre infra-rouge et son spectre de résonance magnétique nucléaire du proton.

Le rendement est de 85%.

Dans un ballon de 50 cm3, on introduit, sous atmosphère d'argon, 1 g de bromo-1 triméthylsilyloxy-2 éthylène (5,3 mmoles) dans 10 cm3 d'éther éthylique anhydre. On refroidit à −70°C puis on ajoute 8 cm3 d'une solution de tertiobutyllithium 1,2 M dans le pentane. On agite pendant 90 minutes à −70°C puis on ajoute 1,19 g de triméthyl-6,10,14 pentadécanone-2 en solution dans 3 cm3 d'éther éthylique anhydre. On laisse la température remonter au voisinage de −15°C et agite pendant 1 heure. On refroidit à −60°C puis ajoute, en 15 minutes, 7 cm3 d'acide chlorhydrique 3N. On agite pendant 30 minutes à 10°C. Après extraction à l'éther, lavage des phases éthérées jusqu'à neutralité, séchage des phases éthérées sur sulfate de magnésium, filtration, évaporation des solvants, on obtient un produit qui est purifié par chromatographie éclair. On obtient ainsi 1,05 g de tétraméthyl-3,7,11,15 hexadécène-2 al qui est caractérisé par son spectre infra-rouge et son spectre de résonance magnétique nucléaire du proton.

Le rendement est de 80%.

Dans un ballon contenant 50 cm3 d'éther anhydre, on introduit, en 5 minutes à 0°C, 0,15 g d'hydrure de lithium et d'aluminium (1 mmole). On agite pendant 10 minutes puis on ajoute 0,3 g de tétraméthyl-3,7,11,15 hexadécène-2 al (1 mmole) en solution dans 5 cm3 d'éther éthylique anhydre. On agite pendant 1 heure à 0°C puis on ajoute 3 cm3 d'acétate d'éthyle et 7 cm3 d'eau saturée de chlorure d'ammonium. On agite pendant 15 minutes puis on extrait à l'éther éthylique. Les phases organiques sont séchées sur sulfate de magnésium. Après filtration et évaporation des solvants, on obtient 0,29 g de tétraméthyl-3,7,11,15 hexadécène-2 ol (ou phytol) qui est caractérisé par son spectre infra-rouge et son spectre de résonance magnétique nucléaire du proton.

## Revendications

1. Nouveau dérivé du nonatriène-1,3,5 caractérisé en ce qu'il répond à la formule générale :

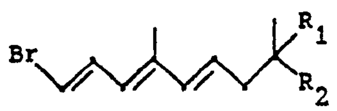

dans laquelle les symboles $R_1$ et $R_2$ forment ensemble avec l'atome de carbone auquel ils sont liés un groupement carbonyl ou bien représentent chacun un radical alkoxy contenant 1 à 4 atomes de carbone ou forment ensemble un radical alkylènedioxy.

2. Procédé de préparation d'un dérivé selon la revendication 1 caractérisé en ce que l'on fait agir un dérivé du butadiène-1,3 de formule générale :

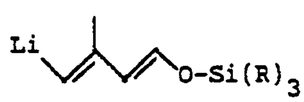

dans laquelle R représente un radical alkyle contenant 1 à 4 atomes de carbone ou un radical phényle, préparé in situ par échange halogène-métal avec le dérivé bromé correspondant, sur un cétoacétal de butanol de formule générale :

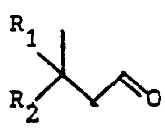

dans laquelle $R_1$ et $R_2$ sont définis comme dans la revendication 1, puis fait agir un halogénure d'halogénométhyltriphénylphosphonium sur l'oxocétal du méthyl-3 oxo-7 octadiène-2,4 al ainsi obtenu.

3. Procédé selon la revendication 2 caractérisé en ce que l'on fait réagir un bromo-1 trialkylsilyloxy-4 butadiène-1,3 sur un dérivé organolithien à une température inférieure à −20°C dans un solvant organique choisi parmi les éthers et les hydrocarbures aliphatiques ou aromatiques, puis fait agir le cétoacétal du butanal, hydrolyse le produit obtenu et isole l'oxocétal du méthyl-3 oxo-7 octadiène-2,4 al.

4. Procédé selon la revendication 2 caractérisé en ce que l'on fait agir un halogénure d'halométhyltriphénylphosphonium sur l'oxocétal du méthyl-3 oxo-7 octadiène-2,4 al en présence d'un alcoolate métallique en opérant dans un solvant organique anhydre choisi parmi les éthers.

5. Procédé de préparation du phytol caractérisé en ce que l'on fait agir un dérivé lithien obtenu par échange halogène-métal avec un produit selon la revendication 1 sur la méthylhepténone pour obtenir l'acétal de l'hydroxy-10 triméthyl-6,10,14 pentadécatétraène-4,6,8,13 one-2 que l'on hydrolyse, déshydrate et réduit pour obtenir la triméthyl-6,10,14 pentadécanone-2 sur laquelle on condense le triméthylsilyloxyvinyllithium, pour obtenir le tétraméthyl-3,7,11,15 hexadécène-2 al que l'on réduit en phytol.

## Ansprüche

1. Nueues 1,3,5-Nonatrien-Derivat, dadurch gekennzeichnet, daß es der allgemeinen Formel :

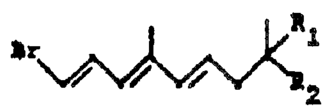

entspricht, in welcher die Symbole $R_1$ und $R_2$ zusammen mit dem Stickstoffatom, an das sie gebunden sind, eine Carbonylgruppe bilden oder jeweils einen Alkoxyrest mit 1 bis 4 Kohlenstoffatomen darstellen oder zusammen einen Alkylendioxyrest bilden.

2. Verfahren zur Herstellung eines Derivats nach Anspruch 1, dadurch gekennzeichnet, daß man ein in situ durch Halogen/Metall-Austausch mit dem entsprechenden Bromderivat hergestelltes 1,3-Butadienderivat der allgemeinen Formel :

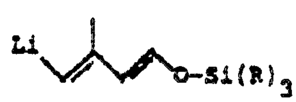

in welcher R einen Alkylrest mit 1 bis 4 Kohlenstoffatomen oder einen Phenylrest darstellt, mit einem Ketoacetal von Butanal der allgemeinen Formel :

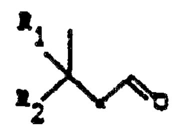

in welcher $R_1$ und $R_2$ die im Anspruch 1 angegebene Bedeutung haben, umsetzt und dann ein Halogenmethyltriphenylphosphoniumhalogenid auf den so erhaltenen 3-methyl-7-oxo-2,4-octadienoxoketal einwirken läßt.

3. Verfahren nach Anspruch 2, dadurch gekennzeichnet, daß man ein 1-Brom-4-trialkylsilyloxy-1,3-butadien mit einer Organo-lithiumverbindung bei einer Temperatur unterhalb −20°C in einem organischen Lösungsmittel, ausgewählt aus den Äthern und den aliphatischen oder aromtischen Kohlenwasserstoffen, umsetzt, dann den Ketoacetal von Butanal einwirken läßt, das erhaltene Produkt hydrolysiert und den Oxoketal des 3-Methyl-7-oxo-2,4-octadienals isoliert.

4. Verfahren nach Anspruch 2, dadurch gekennzeichnet, daß man ein Halogenmethyltriphenylphosphoniumhalogenid auf den Oxoketal des 3-Methyl-7-oxo-2,4-octadienals in Gegenwart eines Metallalkoholats einwirken läßt, wobei man in einem wasserfreien organischen Lösungsmittel, ausgewählt aus den Äthern, arbeitet.

5. Verfahren zur Herstellung von Phytol, dadurch gekennzeichnet, daß man eine Lithiumverbindung, erhalten durch Halogen/Metall-Austausch mit einer Verbindung nach Anspruch 1, auf Methylheptenon unter Bildung des 10-Hydroxy-6,10,14-trimethyl-4,6,8,13-pentadecatetraen-2-on-acetals einwirken läßt, den man hydrolysiert, dehydratisiert und reduziert unter Bildung von 6,10,14-Trimethyl-2-pentadecanon, welches man mit Trimethylsilyloxyvinyllithium unter Bildung von 3,7,11,15-Tetramethyl-2-hexadecenal kondensiert, den man zu Phytol reduziert.

## Claims

1. A new 1,3,5-nonatriene derivative, corresponding to the general formula :

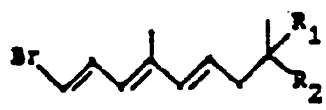

in which the symbols $R_1$ and $R_2$, together with the carbon atom to which they are linked, form a carbonyl group or else each denotes an alkoxy radical containing 1 to 4 carbon atoms or together they form an alkylenedioxy radical.

2. A process for the preparation of a derivative according to claim 1, wherein a 1,3-butadiene derivative of general formula

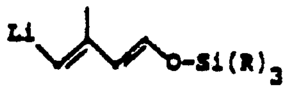

in which R denotes an alkyl radical containing 1 to 4 carbon atoms or a phenyl radical, prepared in situ by halogen-metal exchange with the corresponding bromine derivative, is reacted with a ketoacetal of the butanal of general formula :

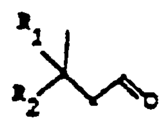

in which $R_1$ and $R_2$ are defined as in claim 1, and a halomethyltriphenylphosphonium halide is then reacted with the oxoketal of the 3-methyl-7-oxo-2,4-octadienal thus obtained.

3. The process according to claim 2, wherein a 1-bromo-4-trialkylsilyloxy-1,3-butadiene is reacted with an organolithium derivative at a temperature below −20°C in an organic solvent chosen from ethers and aliphatic or aromatic hydrocarbons, and the ketoacetal of the butanal is then reacted, the product obtained is hydrolysed and the oxoketal of 3-methyl-7-oxo-2,4-octadienal is isolated.

4. The process according to claim 2, wherein a halomethyltriphenylphosphonium halide is reacted with the oxoketal of 3-methyl-7-oxo-2,4-octadienal in the presence of a metal alcoholate, the operation being carried out in an anhydrous organic solvent chosen from ethers.

5. A process for the preparation of phytol, wherein a lithium derivative obtained by halogen-metal exchange with a product according to claim 1 is reacted with methylheptenone to give the acetal of 10-hydroxy-6,10,14-trimethyl-4,6,8,13-pentadecatetraen-2-one, which is hydrolysed, dehydrated and reduced to give 6,10,14-trimethyl-2-pentadecanone, which is condensed with trimethylsilyloxyvinyllithium, to give 3,7,11,15-tetramethyl-2-hexadecenal, which is reduced to phytol.